Europäisches Patentamt

European Patent Office

Office européen des brevets

(11) Numéro de publication: **0 045 265 B1**

(12) **FASCICULE DE BREVET EUROPEEN**

(45) Date de publication du fascicule du brevet:
**08.10.86**

(21) Numéro de dépôt: **81401214.2**

(22) Date de dépôt: **28.07.81**

(51) Int. Cl.⁴: **G 10 K 11/00, A 61 B 10/00**

(54) Sonde d'échographie à balayage sectoriel mécanique.

(30) Priorité: **29.07.80 FR 8016717**
**29.07.80 FR 8016718**

(43) Date de publication de la demande:
**03.02.82 Bulletin 82/5**

(45) Mention de la délivrance du brevet:
**08.10.86 Bulletin 86/41**

(84) Etats contractants désignés:
**BE CH DE GB IT LI NL SE**

(56) Documents cités:
**WO - A - 79/00371**
**DE - A - 2 925 933**
**FR - A - 2 298 107**
**US - A - 4 181 120**

(73) Titulaire: **Dory, Jacques, 91 rue des Molveaux,**
**F-77450 Coupvray Esblay (FR)**

(72) Inventeur: **Dory, Jacques, 91 rue des Molveaux,**
**F-77450 Coupvray Esblay (FR)**

(74) Mandataire: **Marquer, Francis, 35, Avenue Victor Hugo**
**Résidence Chamfleury, F-78180 Voisins-le-Bretonneux**
**(FR)**

ACTORUM AG

## Description

L'invention se rapporte aux appareils d'échographie médicale comportant un traducteur ultrasonore agencé pour engendrer un faisceau qui effectue un balayage angulaire relativement rapide dans un plan d'examen et est transmis au milieu examiné par un liquide de couplage contenu dans une chambre.

Elle concerne plus particulièrement une sonde d'échographie comportant un traducteur composé, d'une part, d'un boîtier contenant le liquide de couplage et muni d'une fenêtre de passage du faisceau ultrasonore, boîtier dans lequel sont immergés un élément piézoélectrique en forme de disque, un organe moteur électrique et des moyens mécaniques pour provoquer la déviation angulaire du faisceau en fonction de la position angulaire du rotor du moteur; d'autre part, d'un dispositif de repérage continu de la position angulaire du faisceau.

Une telle sonde est destinée à l'échographie en temps réel, dans des applications, telles que la cardiologie, l'ophtalmologie et l'obstétrique, où le nombre d'images par secondes que l'appareil doit afficher est, par exemple, de l'ordre de 25/s à 50/s et où il importe de réaliser une sonde extrêmement légère, compacte, commode d'emploi, peu onéreuse et parfaitement fiable.

Dans le brevet US-A-4 282 879 déposé le 22 Février 1979 et publié le 11 Août 1981, au nom de «Kunii et al», on a décrit une sonde d'échographie comportant un moteur électrique, un élément piézoélectrique en forme de disque et des moyens mécaniques pour provoquer la déviation angulaire du faisceau émis par cet élément piézoélectrique en fonction de la position angulaire du rotor du moteur.

Ces moyens mécaniques comportent une biellette entraînée à une première extrémité à partir de l'arbre du moteur dans un mouvement de révolution décrivant une surface conique autour de l'axe du rotor du moteur, ou premier axe, grâce à un organe de liaison mécanique de ladite biellette audit arbre, ledit organe de liaison mécanique étant axé sur ledit premier axe, des moyens pour réaliser un second axe de rotation fixe du traducteur, ledit premier axe et ledit second axe de rotation se coupant en un centre de rotation et étant perpendiculaires entre eux, et une chape montée pivotante autour d'un troisième axe solidaire du traducteur et perpendiculaire audit second axe de rotation, ladite chape étant solidaire de la seconde extrémité de la biellette.

Selon ledit brevet, la deuxième extrémité de la biellette effectue un mouvement circulaire autour du premier axe et est reliée à la chape par un joint universel, tandis que la première extrémité est située sur le premier axe et reliée à l'arbre du moteur par une glissière réglable. Le moteur est en rotation permanente dans le même sens et l'élément piézoélectrique est monté sur un support massif dont toute la masse est entraînée en oscillation.

Cette solution entraîne des frottements au niveau de la glissière et du joint universel. En outre, elle serait tout à fait inapplicable dans une sonde où le traducteur et ses organes d'entraînement seraient immergés dans un liquide. Enfin, avec une rotation complète du moteur, pendant environ la moitié du cycle de rotation, le traducteur oscille très peu et l'on obtient une non linéarité inacceptable de la variation de la position angulaire du traducteur.

L'invention supprime ces inconvénients au moyen d'une sonde dans laquelle l'arbre du moteur oscille autour d'une position de référence, le second axe de rotation fixe du traducteur et le troisième axe de la chape sont directement solidaires de l'élément piézoélectrique, la biellette est montée fixement sur la chape à sa deuxième extrémité en un point tel que le prolongement de l'axe de la biellette passe par le centre de rotation et que ledit organe de liaison mécanique est agencé pour faire effectuer à la première extrémité, laquelle est décalée par rapport au premier axe, un mouvement circulaire centré sur ledit premier axe et pour autoriser une rotation relative de la biellette autour de son propre axe par rapport audit organe de liaison.

Dans certaines de ces applications, il est intéressant de pouvoir faire travailler l'appareil, soit suivant le mode de représentation dit «type B», dans lequel la trace sur l'écran du faisceau électronique du tube cathodique de visualisation de l'image doit occuper, à chaque instant, une position homothétique de celle du faisceau ultrasonore dans le milieu examiné, soit suivant le mode de représentation dit «TM» ou «temps-mouvement», dans lequel, la sonde étant immobile, on visualise l'évolution en fonction du temps des structures examinées.

Pour réaliser le mode de représentation «type B», il suffit que la sonde fournisse à chaque instant une information de position angulaire du faisceau ultrasonore: on connaît, en effet, des appareils d'échographie munis de moyens pour commander convenablement la déviation du faisceau électronique à partir de cette information mais l'invention ne porte pas sur de tels moyens.

Dans le mode «TM», l'appareil comporte normalement des moyens d'afficher, sur l'écran d'un tube cathodique, une ligne de surbrillance qui peut être déplacée sur l'écran pour l'amener sur une zone de l'image dont on désire observer le mouvement. Lorsque ce repérage a été effectué, le faisceau ultrasonore doit être amené dans la position angulaire correspondante et immobilisé dans cette position et des moyens doivent être prévus pour effectuer le déplacement angulaire approprié du faisceau.

L'invention a encore pour objet un dispositif de repérage de la position angulaire du faisceau ultrasonore, particulièrement apte à coopérer avec lesdits moyens de déviation.

Elle a enfin pour objet un dispositif qui sera associé à la sonde, pour effectuer ledit déplacement angulaire du faisceau approprié au passage de l'appareil d'examen en mode TM, dispositif qui coopère avantageusement avec lesdits moyens de déviation.

L'invention propose de faire comporter à une sonde du genre susvisé, outre une chambre principale qui loge le traducteur, une chambre auxiliaire qui contient un liquide de couplage dans lequel la vitesse de propagation des ultrasons est sensiblement différente de leur vitesse de propagation dans l'eau, et est fermée, dans la direction de transmission du faisceau ultrasonore, par une membrane souple. Ladite chambre auxiliaire est délimitée par ladite membrane principale et par une membrane auxiliaire souple et contient de l'eau, les deux membranes et le liquide de couplage ayant sensiblement la même impédance acoustique que l'eau, et une partie au moins de la surface de ladite membrane principale étant suffisamment souple pour assurer l'équilibre des pressions de liquide dans les deux chambres.

Avec cette disposition, la membrane auxiliaire peut se déformer lors de son application sur la peau sans que cette déformation soit transmise à la membrane principale, laquelle constitue l'interface entre les deux liquides et, par conséquent, aucune distorsion des ondes acoustiques n'accompagne leur réfraction.

D'autres particularités, ainsi que les avantages de l'invention, apparaîtront clairement à la lumière de la description ci-après.

Au dessin annexé:

La figure 1 est une vue schématique, en coupe partielle par un plan axial, d'une sonde conforme à un mode d'exécution préféré de l'invention;

La figure 2 représente schématiquement, vu en perspective, le mécanisme d'entraînement du traducteur;

La figure 3 représente la liaison entre la biellette et son organe d'entraînement;

La figure 4 est une vue de détail du traducteur et de la chape qui provoque son oscillation;

La figure 5 représente le dispositif magnétique de rappel du moteur dans une position de référence;

La figure 6 est un schéma des circuits du dispositif de repérage de la position angulaire du faisceau et du dispositif qui permet le déplacement angulaire du faisceau approprié au passage en mode TM;

La figure 7 représente une variante d'exécution du pont de mesure de la vitesse angulaire du moteur, qui comporte ledit dispositif de repérage;

La figure 8 est une vue en coupe d'une sonde à deux membranes conforme à un mode d'exécution préféré de l'invention;

La figure 9 est une vue en perspective de l'extrémité de ladite sonde, la membrane extérieure étant enlevée, et

La figure 10 est une coupe suivant X–X de la figure 8.

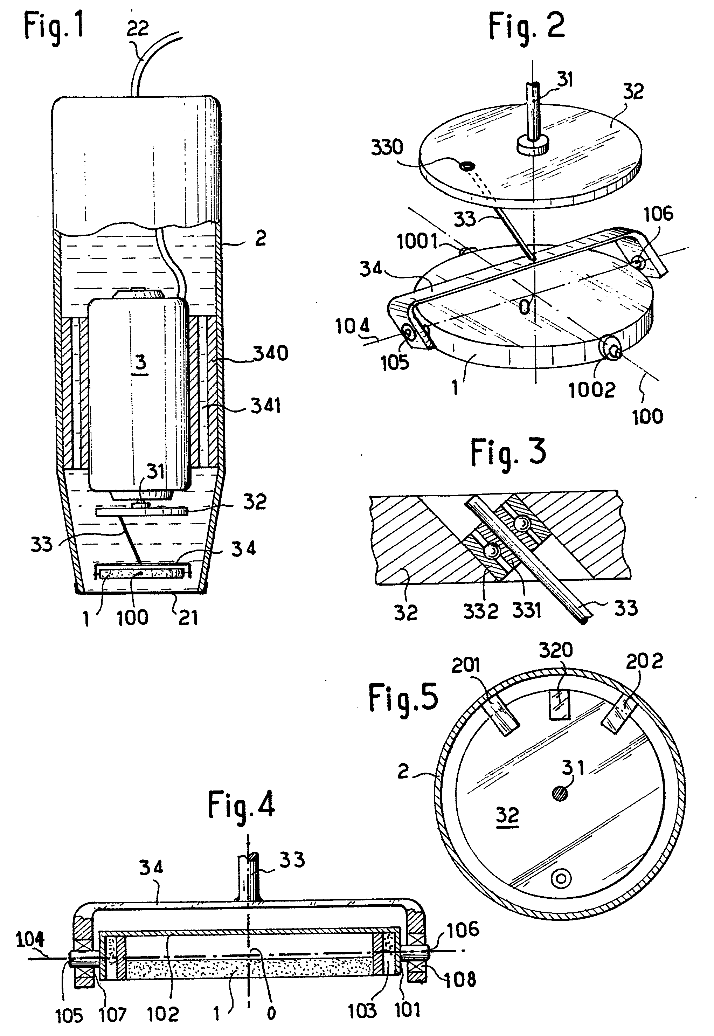

Aux figures 1 et 2, on a représenté un traducteur constitué d'une pastille de céramique piézoélectrique 1 logée au voisinage d'une membrane 21 transparente aux ultrasons, dans un boîtier 2 qui contient un liquide de couplage. Ce dernier est de préférence du type dans lequel la vitesse de propagation des ultrasons est plus faible que leur vitesse de propagation dans l'eau, par exemple certains liquides fluorés.

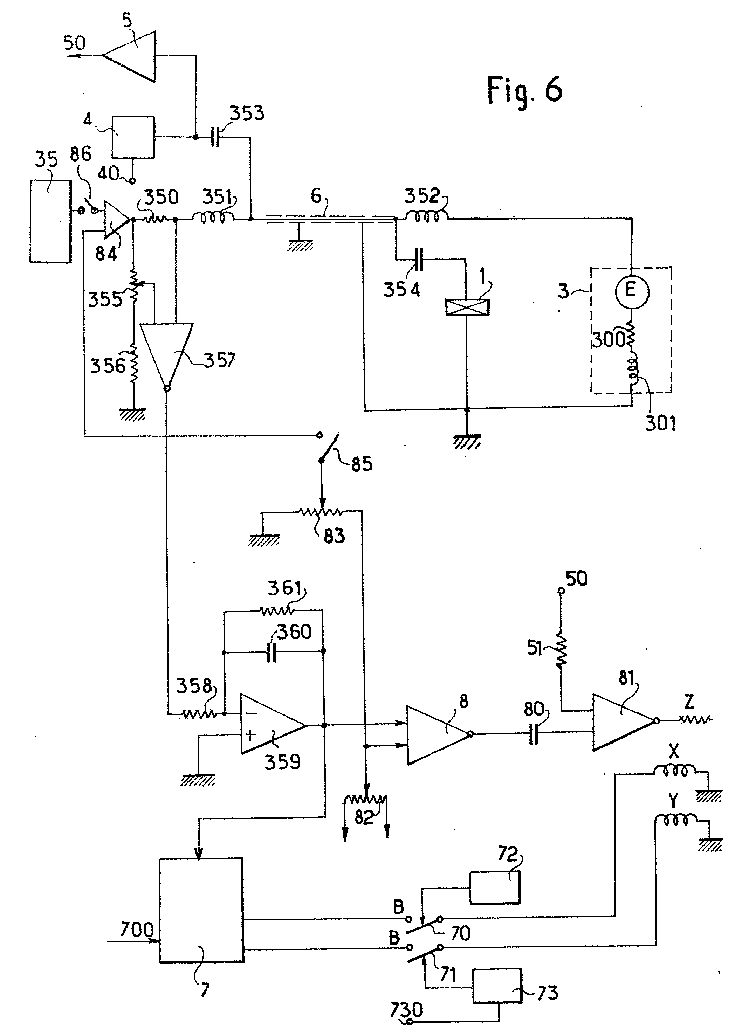

Un tel liquide est inerte, ce qui permet d'utiliser un moteur immergé. Le boîtier 2 a la forme d'un cylindre allongé terminé par une partie tronconique dont l'extrémité est fermée par la membrane 21. On a représenté en 22 un câble de liaison électrique de la sonde ainsi constituée et les circuits d'émission et de réception que comporte l'appareil d'examen. On expliquera en se référant à la figure 6, par quels moyens, propres à l'invention, l'utilisation d'un câble unique a été rendue possible.

Un moteur 3 est fixé au boîtier au moyen d'un manchon 34 percé de canaux 340 qui permettent la circulation du liquide de couplage.

Le traducteur 1 est entraîné en oscillation autour d'un axe 100, perpendiculaire au plan de la figure 1, au moyen du moteur 3 à très faible inertie, dont l'arbre 31 est disposé suivant l'axe du boîtier 2 et qui oscille par exemple de $\pm 20°$ autour d'une position de référence définie par un système magnétique ou élastique, comme on l'expliquera en se référant à la figure 5. Sur l'arbre 31 est monté un disque 32 perpendiculaire à l'axe du boîtier 1. Une biellette 33 est liée au disque 32, de la manière qui sera expliquée en se référant à la figure 3, en un point périphérique 330 du disque 32; la biellette 33 coopère avec une chape 34 fixée à la pastille 1, de la manière qui sera expliquée en se référant à la figure 4.

A la figure 3, on a représenté la liaison entre le disque 32 et la biellette 33. On voit que la tête 331 de cette dernière est montée dans un roulement 332, si bien que le seul mouvement que puisse exécuter la biellette 33, par rapport au disque 32, est une rotation sur elle-même autour de son propre axe, à l'exclusion de toute translation parallèle à son axe.

La biellette 33 est fixée à la chape 34, en un point tel que l'axe de ladite biellette passe par le centre 0 du disque 1. La biellette effectue donc un mouvement de révolution décrivant un cône autour de l'axe de symétrie perpendiculaire au plan du traducteur, lequel axe passe par 0 et se confond avec l'axe de l'arbre 31. Le demi-angle au sommet du cône sera par exemple de l'ordre de 45°.

La pastille de céramique 1 est fixée à la partie inférieure d'une bague 101 elle-même solidaire d'une coupelle de support 102. Les éléments 101 et 102 peuvent être en matière plastique et l'intervalle 103 entre la paroi latérale de la coupelle et la bague 101 sera avantageusement rempli de colle. Sur la paroi latérale de la coupelle sont montés, aux deux extrémités respectives du diamètre 104 (voir figure 2) de la pastille 1 perpendiculaire à l'axe 100, deux tourillons 105–106 (figures 2 et 4) qui coopèrent respectivement avec des roulements 107–108 dont la cage extérieure est solidarisée, comme le montre la figure 4, aux extrémités de la chape 34.

Le seul mouvement qui puisse exécuter le disque 1 est une oscillation alternative autour de l'axe 100. Ce dernier est défini par deux tourillons 1001 et 1002 également fixés à la coupelle 102 et coopérant avec des paliers, non figurés, fixés au boîtier 2. L'axe 104 oscille lui-même dans le plan passant par 0 et perpendiculaire à l'axe 100 et la chape 34 ne peut elle-même qu'osciller autour des axes mobiles 104 et 33. En définitive, l'oscillation de la pastille 1, de l'ordre de $\pm 20$ à 25° par exemple, est obtenue uniquement à partir de mouvements de rotation, avec des organes ne présentant aucun jeu ou glissement. Il est évident que le disque 32 pourra être remplacé par une manivelle.

Outre la fiabilité qui en résulte, ce dispositif présente l'avantage qu'il existe une liaison univoque entre les positions angulaires de l'arbre 31 et de la pastille 1, ce qui facilite le repérage de la déviation angulaire du faisceau émis. On notera, par ailleurs, que le dispositif est entièrement distribué autour de l'axe du moteur, ce qui permet la réalisation d'une sonde coaxiale, d'emploi particulièrement commode, notamment en cardiologie.

A la figure 5, on a représenté le disque 32 en vue de dessus et le boîtier 2 en coupe transversale par un plan parallèle au disque 32. On voit que deux petits aimants 201 et 202 sont montés radialement sur la face périphérique intérieure du boîtier 2, un peu au-dessus de la surface du disque 32. Ce dernier porte de son côté un aimant radial 320. Ces trois aimants sont aimantés dans des directions tangentielles par rapport à la périphérie du disque et disposés de manière telle que l'aimant 320 présente des surfaces polaires respectivement en regard de surfaces polaires des aimants 201 et 202, les pôles en regard étant de même signe. Il en résulte que, par effet de répulsion, l'aimant 320 prendra, en l'absence d'entraînement par le moteur, une position d'équilibre entre les positions des aimants 201 et 202, à égale distance de ceux-ci s'ils sont identiques. L'angle que font entre elles les directions radiales des aimants 201 et 202 devra évidemment être un peu supérieur à l'angle d'oscillation de l'arbre 31. On définit ainsi une position angulaire de référence de l'arbre 31.

On notera que ce dispositif de rappel magnétique (non représenté à la figure 2 pour simplifier) pourrait, non seulement être monté sur un autre organe rotatif solidaire de l'arbre du moteur, mais encore être remplacé par un dispositif de rappel élastique par ressort, ou par tout autre moyen approprié. Il est toutefois particulièrement simple et fiable. De même, le moteur pourrait être remplacé par un dispositif galvanométrique.

La membrane 21 sera avantageusement, dans le cas où le liquide de couplage contenu dans le boîtier est du type mentionné plus haut, de nature souple et associée à une deuxième membrane souple délimitant avec la première une chambre auxiliaire contenant de l'eau, comme décrit dans la demande de brevet français déposée par le Demandeur, pour: «Sonde d'échographie à balayage sectoriel comportant deux liquides de couplage» et publié pour la première fois sous le No FR-A-2 487 664 le 5 Février 1982 (publication de la délivrance le 10 Novembre 1983). Toutefois, l'on pourra, dans certains cas, se passer de cette disposition et se contenter de garnir intérieurement la membrane 21 d'une matière absorbant les ultrasons, ayant pour effet de réduire les réflexions parasites au niveau de la membrane.

A la figure 6, on a représenté un générateur 35 de dents de scie ou de signaux rectangulaires ayant par exemple une fréquence de récurrence de 100 Hz, destiné à fournir, à travers un amplificateur 84, une résistance 350 et des inductances 351 et 352 (par exemple de 100 mH), ayant à la fréquence de travail, une impédance élevée par rapport à celle des résistances du pont et reliées entre elles par un câble coaxial 6, le courant d'excitation du moteur 3. Celui-ci a été symbolisé par une force contre-électromotrice E, (qui sera proportionnelle à la vitesse de rotation du moteur) en série avec une résistance 300 et une inductance 301.

On a représenté en 4 l'émetteur d'impulsions (ayant une fréquence de récurrence par exemple comprise entre 2 et 5 MHz) et en 5 le récepteur que comporte l'appareil d'examen. Ces organes sont reliés à l'inductance 351, un condensateur 353, tandis que l'inductance 352 et reliée au traducteur 1 par un condensateur 354.

La sortie du générateur 35 attaque, par l'intermédiaire d'un commutateur 86, une première entrée de l'amplificateur 84, dont la sortie est reliée à la masse par l'intermédiaire d'un potentiomètre 355, en série avec une résistance 356. Un amplificateur différentiel 357 a sa sortie reliée, par l'intermédiaire d'une résistance 358, à l'entrée négative d'un amplificateur opérationnel 359 dont l'entrée positive est reliée à la masse. La sortie de l'amplificateur 359 est reliée à son entrée négative par l'intermédiaire d'un condensateur 360 sur lequel une résistance 361 est branchée en parallèle. La sortie de l'amplificateur 359 est, par ailleurs, reliée à l'électrode de commande d'un tube cathodique par l'intermédiaire d'un circuit comprenant un amplificateur différentiel 8, un condensateur 80 et un amplificateur 81.

Les entrées de l'amplificateur 357 sont connectées au curseur du potentiomètre 355 et, respectivement, au point commun entre la résistance 350 et l'inductance 351.

Le courant à basse fréquence qui circule dans la résistance 350, les inductances 351 et 352 et les enroulements rotoriques du moteur, n'est évidemment transmis ni aux organes émetteur et récepteur 4 et 5 (il est bloqué par le condensateur 353), ni au traducteur 1 (condensateur 354). De même, le courant à haute fréquence qui est transmis de l'émetteur 4 au traducteur 1 et de ce dernier au récepteur 5, par les condensateurs 353 et 354 ne perturbe pas (selfs de choc 351–352) le trajet du courant à basse fréquence susvisé. On voit que, dans le montage décrit, il en résulte finalement que la liaison entre le traducteur 1 et les circuits extérieurs peut se faire au moyen d'un câble coaxial unique.

Le montage décrit sert à fournir, à la sortie de l'amplificateur opérationnel 359 qui fonctionne en intégrateur, une tension proportionnelle à la déviation angulaire θ du moteur (donc du traducteur) par rapport à une position de référence, comme on va maintenant l'expliquer.

Les résistances en série 355 et 356 d'une part, la résistance 350 en série avec l'impédance du moteur d'autre part, forment un pont. L'amplificateur 357 est branché selon l'une des diagonales du pont. Les selfs 351 et 352 ayant une impédance négligeable à 100 Hz. A l'équilibre du pont, obtenu à l'arrêt du moteur, en ajustant convenablement le potentiomètre 355, il n'existe aucune tension aux bornes de l'amplificateur 357. La tension auxdites bornes est finalement, en fonctionnement, proportionnelle à la force contre-électromotrice E, elle-même proportionnelle à la vitesse du moteur. Par intégration, on obtient donc, à la sortie de l'amplificateur 359, une tension proportionnelle à la déviation angulaire θ.

Pour obtenir un repérage absolu de la position angulaire, il faut, d'une part, qu'une position mécanique de référence de l'ensemble oscillant soit définie et, d'autre part, que la tension de sortie de l'intégrateur 359 soit nulle en l'absence du signal d'entrée (c'est-à-dire lorsque le moteur est bloqué).

Le premier résultat est obtenu au moyen du système magnétique déjà décrit, qui rappelle l'arbre du moteur dans une position bien définie en l'absence de courant d'excitation.

Le second résultat est obtenu au moyen du condensateur 360 et de la résistance 361. Le condensateur 360 se charge en présence d'une tension à l'entrée de l'amplificateur. En l'absence d'une telle tension, il se décharge progressivement à travers la résistance 361, si bien que la tension de sortie de l'amplificateur est, au bout d'un certain temps, remise à zéro.

L'information de déviation angulaire est appliquée à un dispositif 7, connu en soi, qui reçoit par ailleurs, à son entrée 70, les signaux de synchronisation d'émission (provenant de la borne 40 de l'émetteur 4) et fournit, sur les organes de déviation X et Y du tube cathodique, des tensions en dents de scie d'amplitude instantanée proportionnelle à $t \sin \theta$ et à $t \cos \theta$ respectivement, t étant le temps. Deux commutateurs 71 permettent de substituer à ces tensions des tensions fournies par deux générateurs de balayage 72–73 respectivement. Le générateur 72 fournit un balayage lent de l'écran et le générateur 73, un balayage de type classique en dents de scie synchrones de l'émission, la borne 730 étant, à cet effet, reliée à la borne 40.

La sortie de l'amplificateur-intégrateur 359 est appliquée par ailleurs à l'amplificateur différentiel 8, monté en comparateur, dont la sortie attaque, par l'intermédiaire du condensateur 80, l'amplificateur 81 qui attaque lui-même l'électrode de commande de luminosité Z du tube cathodique. L'autre entrée de l'amplificateur 81 est reliée à la sortie 50 du récepteur 5 par l'intermédiaire d'une résistance 51, tandis que l'autre entrée de l'amplificateur 8 est reliée au curseur d'un potentiomètre 82 aux bornes duquel est appliquée une tension continue. Ledit curseur est, par ailleurs, relié à la masse par l'intermédiaire d'un potentiomètre 83, dont le curseur est relié à une deuxième entrée de l'amplificateur 84 par l'intermédiaire d'un commutateur 85.

Lorsque le commutateur 86 est fermé et le commutateur 85 ouvert, la tension appliquée au moteur est celle fournie par le générateur 35 et le moteur effectue un mouvement oscillant comme indiqué plus haut. A ce moment, les commutateurs 70 et 71 sont dans la position indiquée «B» au dessin, si bien que l'écran du tube cathodique est balayé selon le mode B.

Le comparateur 8 compare la tension continue $K\theta_0$ prélevée sur le potentiomètre 82 à la tension K prélevée à la sortie de l'intégrateur 359 et superpose, à l'information vidéo appliquée à l'électrode Z, un signal rectangulaire à chaque fois que $K = K_0$. Il en résulte que, pour une position angulaire déterminée du faisceau ultrasonore, une ligne de surbrillance est affichée sur l'écran.

Lorsqu'on désire observer, selon le mode TM, une structure particulière en mouvement visualisée sur l'écran, on règle le potentiomètre 82 jusqu'à amener la ligne de surbrillance sur ladite structure.

A ce moment, l'on commute les commutateurs 70–71–85 et 86. Il en résulte, d'une part, que l'écran est balayé, de façon connue en soi, selon le mode de représentation TM, d'autre part, que le moteur n'est plus excité que par une tension continue $KK_1\theta_0$, $K_1$ étant un coefficient de proportionnalité qui dépend du réglage du potentiomètre 83. Cette tension engendre un couple moteur qui fait tourner le rotor du moteur d'un certain angle, jusqu'à équilibre avec le couple de rappel déterminé par le dispositif magnétique décrit ci-dessus. Le réglage du potentiomètre 83 est effectué de façon telle que cette position d'équilibre corresponde précisément à celle de la ligne de surbrillance définie ci-dessus. Il en résulte que la sonde s'immobilise dans ladite position.

On appréciera que le passage du mode B en mode TM est ainsi obtenu de manière simple, grâce au fait que le dispositif d'entraînement du traducteur possède un couple de rappel dans une position de référence.

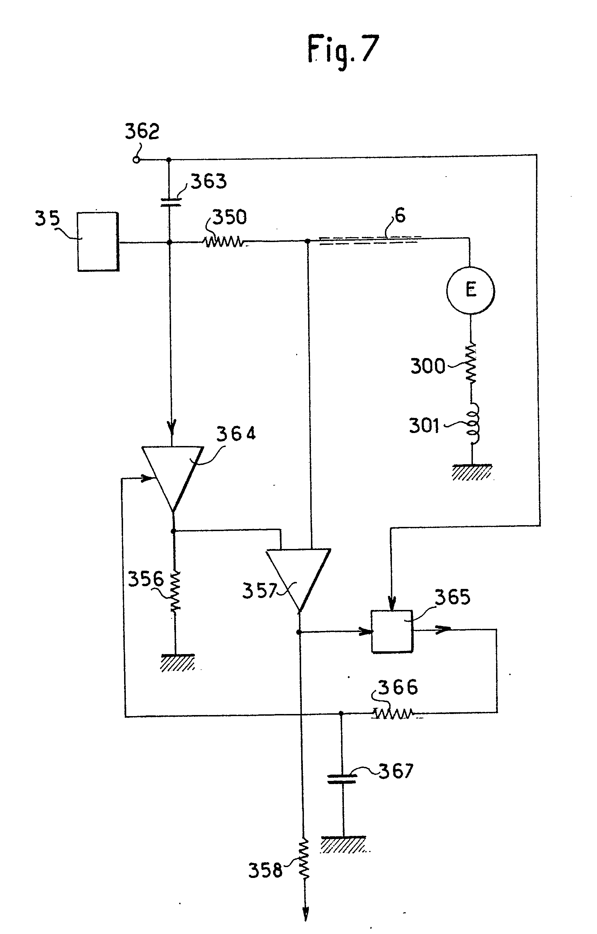

A la figure 7, on a représenté une variante du pont de mesure de la vitesse de rotation du moteur qui fait partie du circuit de la figure 6. La résistance 355 de la figure 6 est remplacée, dans cette variante, par un amplificateur à gain variable, en série avec la résistance 356, et dont le gain est commandé par une tension continue issue d'un démodulateur synchrone 365, après filtrage par une cellule à résistance 366 et capacité 367. Le démodulateur synchrone 365 reçoit, d'une part la sortie de l'amplificateur différentiel 357 du pont, d'autre part, une tension à 1000 Hz par exemple appliquée à la borne 362. Cette dernière tension, de préférence de fréquence suffisamment élevée pour ne pas entraîner le moteur, est également appliquée, par l'intermédiaire d'un condensateur 363, au point commun entre la résistance 350 et l'entrée de l'amplificateur 364. Les inductances 351 à 352, qui ne jouent aucun rôle dans l'équilibre du pont, n'ont pas été représentées pour simplifier le dessin. Il est évident qu'à 1000 Hz, le pont doit être équilibré, même si le moteur est en marche, puisqu'il n'engendre aucune force contre-électromotrice à cette fréquence.

Le démodulateur 365, de type connu en soi, engendre une tension continue proportionnelle en amplitude et en signe au signal résiduel à 1000 Hz qui sort de l'amplificateur 357 lorsque le pont n'est pas équilibré à 1000 Hz (par exemple, à cause d'un changement de sonde). Cette tension continue modifie le gain de l'amplificateur 364 dans un sens propre à réaliser le ré-équilibrage automatique du pont.

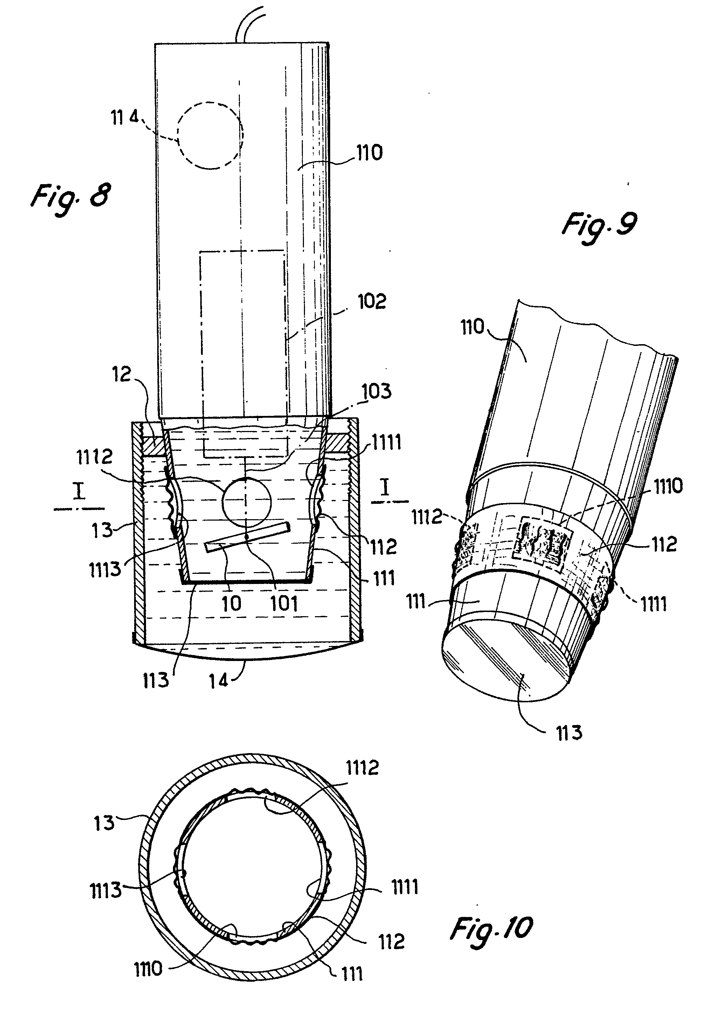

A la figure 8, l'on n'a représenté pour simplifier, que le traducteur piézoélectrique 10, qui effectue un mouvement d'oscillation autour d'un axe 101 perpendiculaire au plan de la figure, sous la commande d'un moteur électrique, symbolisé par un rectangle en trait mixte 102 et d'un mécanisme de liaison, symbolisé par un trait mixte 103. Ces organes sont logés dans un boîtier en métal ou en matière plastique, comportant une partie cylindrique 110 prolongée par une partie tronconique 111 ouverte à sa base, raccordées entre elles de façon amovible. La partie tronconique est ceinturée, au voinage de sa base, par une bande 112, constituée d'une feuille très mince et très souple, par exemple en caoutchouc naturel, collée au boîtier, et faisant face à cette bande, le boîtier 111 comporte des fenêtres 1110 à 1113, figures 9 et 10.

La base de la partie 111 du boîtier est fermée par une membrane 113 fixée par collage ou munie d'un rebord annulaire et emmanchée à force. Cette membrane est constituée d'une matière relativement souple et ayant une impédance acoustique voisine de celle de l'eau, telle qu'un caoutchouc naturel, un élastomère siliconé, un polyéthylène ou la matière plastique commercialisée sous la marque «Dutral» déposée par la firme Montedison (Polyéthylène-Propylène).

Dans ce dernier cas, la membrane présente une rigidité suffisante pour pouvoir être conformée et conserver sa forme en l'absence de contrainte, ce qui permet, au lieu de la tendre à plat, de lui donner une courbure prédéterminée.

Un anneau fileté 12 fixé à la partie 111 au voisinage de son raccordement avec la partie 110 du boîtier, coopère avec un manchon en matière rigide 13 lui-même fileté intérieurement à son extrémité supérieure. A la base de ce manchon, on a collé, ou fixé par pincement ou de toute autre manière, une membrane 14 en matière souple ayant une impédance acoustique voisine de celle de l'eau, par exemple un caoutchouc naturel, un élastomère siliconé ou un polyuréthane.

La chambre fermée délimitée par le boîtier 110, 111, les portions de la bande 112 correspondant aux fenêtres 1110 à 1113 et la membrane 113 contient un liquide fluoré dont la densité est, par exemple, voisine du double de celle de l'eau, alors que la vitesse de propagation des ultrasons y est par exemple moitié de leur vitesse de propagation dans l'eau; il en résulte que l'impédance acoustique d'un tel liquide est sensiblement la même que celle de l'eau.

La chambre fermée délimitée par l'anneau 12, le manchon 13 et la membrane 14 contient de l'eau.

Les portions de la bande 112 correspondant aux fenêtres 1110 à 1113, extrêmement souples, se déforment pour assurer un équilibre permanent de pression entre les deux chambres. Il en résulte que la déformation de la membrane 14 lorsqu'on l'applique sur la peau du patient n'entraîne aucune déformation de la membrane 113.

Selon la forme de la surface d'application, la membrane 113 sera avantageusement plus ou moins bombée: ce réglage est obtenu en vissant plus ou moins l'anneau 12.

Dans le liquide spécial contenu dans la chambre principale, on immerge avantageusement une cavité remplie d'air 114 fermée par une membrane souple et dont les variations de volume en fonction de la température du liquide compenseront les variations de la pression exercée par celui-ci.

Comme on l'a indiqué ci-dessus, la membrane 113 peut recevoir une certaine courbure et constituer par exemple, avec le liquide qu'elle contient, une lentille convergente servant à la focali-sation du faisceau ultrasonore. Bien entendu, il serait également possible d'accoler une lentille acoustique séparée, de type connu en soi, à la membrane 113.

**Revendications**

1. Sonde d'échographie comportant: un traducteur comprenant un boîtier (2) contenant un liquide de couplage et muni d'une fenêtre de passage du faisceau ultrasonore, dans lequel sont ménagés un élément piézoélectrique en forme de disque (1); un organe moteur électrique (3) dont l'arbre est monté rotatif autour d'un premier axe (31) et des moyens mécaniques pour provoquer la déviation angulaire du faisceau en fonction de la position angulaire de l'arbre du moteur, lesdits moyens mécaniques comportant:

– une biellette (33) entraînée à une première extrémité (330) à partir dudit arbre dans un mouvement de révolution décrivant une surface conique autour dudit premier axe (31) grâce à un organe de liaison mécanique (32) de ladite biellette (33) audit arbre, ledit organe de liaison mécanique (32) étant axé sur le premier axe;

– des moyens (1001–1002) pour réaliser un second axe de rotation fixe (100) du traducteur, ledit premier axe (31) et ledit second axe de rotation (100) se coupant en un centre de rotation (0) et étant perpendiculaires entre eux;

– et une chape (34) montée pivotante autour d'un troisième axe (104) solidaire du traducteur passant par ledit centre de rotation (0), et perpendiculaire audit second axe de rotation (100), ladite chape étant solidaire de la seconde extrémité de la biellette,

sonde dans laquelle l'arbre du moteur oscille autour d'une position de référence (320, figure 5), le second axe de rotation fixe (100) du traducteur et le troisième axe (104) de la chape (34) sont directement solidaires de l'élément piézoélectrique (1), la biellette (33) est montée fixement sur la chape (34) à sa deuxième extrémité en un point tel que le prolongement de l'axe de la biellette passe par le centre de rotation (0) et que ledit organe de liaison mécanique (32) est agencé pour faire effectuer à la première extrémité (330), laquelle est décalée par rapport au premier axe (31), un mouvement circulaire centré sur ledit premier axe (31) est pour autoriser une rotation relative de la biellette (33) autour de son propre axe par rapport audit organe de liaison (32).

2. Sonde selon la revendication 1, caractérisée en ce que ledit organe de liaison mécanique comprend un disque (32) perpendiculaire audit arbre (31) qui est solidaire dudit arbre (31) et un roulement (331–332) dans lequel est montée la première extrémité (330) de la biellette (33), ledit disque (32) étant muni d'un aimant (320) qui coopère avec des aimants fixes (201–202) de manière à constituer un couple de rappel de l'équipage mobile vers une position d'équilibre.

3. Sonde selon la revendication 1 ou 2, comportant en outre un dispositif de repérage continu de la position du faisceau ultrasonore, caractérisée en ce que ledit dispositif de repérage comporte un pont de mesure (355–356–350, E) de la force contre-électromotrice engendrée par ledit organe moteur, lequel possède une position ou mécanique de référence définie par un couple de rappel et des moyens (39) d'intégrer le signal de mesure obtenu, lesdits moyens étant agencés (résistance 361 – condensateur 360) pour que leur signal de sortie soit nul lorsque ledit organe moteur n'est pas en mouvement.

4. Sonde selon la revendication 3, caractérisée par des moyens (condensateurs 353 et 354) d'empêcher la transmission du courant à basse fréquence (générateur 35) d'excitation du moteur vers les organes émetteur (4) et récepteur (5) de l'appareil d'examen et vers le traducteur (1) et par des moyens (inductances 351 et 352) d'empêcher la transmission du courant à haute fréquence d'émission et de réception vers ledit pont de mesure, afin de permettre la liaison de la sonde aux organes extérieurs au moyen d'un câble coaxial unique (6).

5. Sonde selon l'une des revendications 3 ou 4, caractérisée en ce que ledit pont de mesure comprend un amplificateur à gain variable (364) commandé par un démodulateur synchrone (365) qui reçoit d'une part, ledit signal de sortie du pont, d'autre part, une tension de fréquence suffisamment élevée pour ne pas être susceptible d'entraîner le moteur, ladite tension étant appliquée au pont.

6. Sonde selon l'une des revendications 3 à 5, comportant des moyens (8–82) de prélever une tension proportionnelle audit signal de sortie, de la comparer à une tension continue réglable et d'engendrer, lors de la coïncidence, un signal de commande de surbrillance destiné au tube cathodique de l'appareil d'examen et des moyens (83–85–86) d'appliquer au moteur une tension proportionnelle à ladite tension continue, qui se substitue au courant normal d'excitation du moteur pour provoquer son arrêt dans une position angulaire qui correspond à celle définie par ledit signal de commande de surbrillance.

7. Sonde selon la revendication 1, agencée pour engendrer un faisceau d'ultrasons effectuant un balayage angulaire rapide dans le plan d'examen, ledit faisceau étant transmis au milieu examiné par un liquide de couplage contenu dans une chambre, à travers une membrane (113) transparente aux ultrasons qui forme une paroi de cette chambre, ledit liquide de couplage étant de nature telle que la vitesse de propagation des ultrasons y soit sensiblement différente de leur vitesse de propagation dans l'eau, caractérisée par une chambre auxiliaire, délimitée par ladite membrane principale (112–113) et par une membrane auxiliaire souple (14) et contenant de l'eau, les deux membranes et le liquide de couplage ayant sensiblement la même impédance acoustique que l'eau, et une partie au moins (122) de la surface de ladite membrane principale (112–113) étant suffisamment souple pour assurer l'équilibre des pressions de liquide dans les deux chambres.

8. Sonde selon la revendication 7, caractérisée en ce que la membrane principale comprend une première partie (113) qui ferme le fond de la chambre principale et une seconde partie constituée par des portions d'une bande très souple (112) situées en regard de fenêtres (1110 à 1113) ménagées dans la paroi latérale de ladite chambre principale.

9. Sonde selon la revendication 7 ou 8, caractérisée en ce que la portion (113) de membrane principale qui ferme le fond de la chambre principale est conformée de manière à constituer une lentille convergente.

10. Sonde selon l'une des revendications 7 à 9, caractérisée en ce que la paroi latérale de la chambre auxiliaire est constituée d'un manchon (13) fileté intérieurement à son extrémité supérieure et coopérant avec un anneau fileté (12) qui permet le réglage de la pression d'eau dans la chambre auxiliaire.

11. Sonde selon l'une des revendications 7 à 10, caractérisée par une cavité fermée (114) remplie d'air agencée pour que sa variation de volume en fonction de la température entraîne une variation en sens inverse du volume de liquide dans la chambre principale.

**Claims**

1. Probe for echography comprising: a transducer consisting of a casing (2) containing a coupling liquid and fitted with a window for the passage of the ultrasonic beam, in which are provided a piezoelectric element in the form of a disc (1); an electric drive unit (3) whose shaft is mounted for rotation about a first axis (31) and mechanical means to produce the angular deviation of the beam according to the angular position of the rotor of the motor, said means comprising:

– a link (33) driven at a first end (330) from said shaft into a rotation movement defining a conical surface around said first axis (31) by a mechanical unit (32) for connecting said link (33) to said shaft, said mechanical connection unit (32) being centered on the first axis;

– means (1001–1002) for producing a second fixed axis of rotation (100) in the transducer, said first axis (31) and said second axis of rotation (100) intersecting at a center of rotation (0) and being mutually perpendicular;

– and a yoke (34) mounted for pivoting around a third axis (104) integral with the transducer passing through said center of rotation (0) and perpendicular to said second axis of rotation (100), said yoke being integral with the second end of the link;

probe in which the motor shaft oscillates around a reference position (320, figure 5), the second fixed axis of rotation (100) of the transducer and the third axis (104) of the yoke (34) are directly integral with the piezoelectric element (1) the link (33) is fixedly mounted on the yoke (34) at its second end at a point such that the extension of the axis of the link passes through the center of rotation (0) and said mechanical connection unit (32) is adapted to cause the first end (330), staggered with respect to the first axis (31), to effect a circular movement centered on said first axis (31) and to allow a relative rotation of the link (33) about its own axis with respect to said connection unit (32).

2. Probe according to claim 1, characterized by the fact that the said mechanical link comprises a disc (32) perpendicular to the said shaft (31) which is integral with said shaft (31) and a bearing (331–332) in which is fitted the first end (330) of the link (33), the said disc (32) being provided with a magnet (320) which cooperates with fixed magnets (201–202) so as to form a torque bringing back the moving assembly to a position of equilibrium.

3. Probe according to claim 1 or 2, comprising in addition a system for continuously indicating the position of the ultrasonic beam, characterized by the fact that the said indicating system comprises a bridge (355–356–350, E) for measuring the back EMF generated by the said drive unit, which possesses a reference position or mechanic defined by a restoring torque and means (39) for integrating the measurement signal obtained, the said means being arranged (resistor 361 – capacitor 360) such that their output signal is zero when the said drive unit is at rest.

4. Probe according to claim 3, characterized by means (capacitors 353 and 354) for preventing transmission of the low frequency motor excitation current (generator 35) to the emitter (4) and receiver (5) units of the inspection equipment and to the transducer (1) and by means (inductances 351 and 352) for preventing transmission of the high frequency emission and reception current to the said measurement bridge, in order to allow the probe to be connected to the external equipment by means of a single coaxial cable (6).

5. Probe according to anyone of claim 3 or 4, characterized by the fact that the said measurement bridge comprises a variable gain amplifier (364) controlled by a synchronuous demodulator (365) to which is fed, on the one hand, the said bridge output signal and, on the other hand, a voltage of sufficiently high frequency as to be incapable of driving the motor, the said voltage being applied to the bridge.

6. Probe according to anyone of claims 3 or 5, comprising means (8–82) for tapping a voltage proportional to the said output signal, comparing it to an adjustable direct voltage and generating, when these coincide, a brightening signal for the CRT of the inspection equipment and means (83–85–86) for applying to the motor a voltage proportional to the said direct voltage, which replaces the normal motor excitation current in order to cause it to stop in an angular position that corresponds to the one defined by the said brightening signal.

7. Probe according to claim 1, arranged so as to generate an ultrasonic beam describing a rapid angular scan in the plane of inspection, the said beam being transmitted to the medium under examination by a coupling liquid contained in a chamber, through a membrane (113) transparent to the ultrasonic waves which forms one wall of this chamber, the said coupling liquid being such that the speed of propagation of the ultrasonic waves in it is appreciably different from the speed of propagation in water, characterized by an auxiliary chamber, bounded by the said principal membrane (112–113) and by an auxiliary flexible membrane (14) and containing water, the two membranes and the coupling liquid having virtually the same acoustic impedance as water, and at least one part (122) of the surface of the said principal membrane (112–113) being sufficiently flexible to ensure equilibrium of the liquid pressures in the two chambers.

8. Probe according to claim 7, characterized by the fact that the principal membrane comprises a first part (113) which closes the bottom of the principal chamber and a second part consisting of portions of a highly flexible strip (112) located opposite windows (1110 to 1113) arranged in the sidewall of the said principal chamber.

9. Probe according to claim 7 or 8, characterized by the fact that the portion (113) of the principal membrane which closes the bottom of the principal chamber is shaped so as to form a converging lens.

10. Probe according to anyone of claims 7 to 9, characterized by the fact that the sidewall of the auxiliary chamber consists of a sleeve (13) tapped at its upper end and cooperating with a threaded bush (12), whereby the water pressure in the auxiliary chamber can be adjusted.

11. Probe according to anyone of claims 7 to 10, characterized by a closed cavity (114) filled with air arranged such that its change in volume according to the temperature causes an opposite change in the volume of liquid in the principal chamber.

**Patentansprüche**

1. Sonde für Echographie mit: einem Messwandler, der ein Gehäuse (2) mit Kupplungsflüssigkeit und ein Durchlassfenster für den Ultraschallstrahl besitzt, in dem ein piezoelektrisches Element in Form einer Scheibe (1) enthalten ist; ein elektrisches Antriebsorgan (3), dessen Welle um eine erste Achse (31) drehbar montiert ist und mechanische Mittel, um die Winkelverschiebung des Strahls je nach der Winkelstellung der Motorwelle zu bewirken, wobei besagte mechanische Mittel enthalten:

– einen Schwingarm (33), der an einem ersten Ende (330) von besagter Welle aus in eine Drehbewegung versetzt wird, die um besagte erste Achse (31) eine konische Fläche beschreibt, mittels eines Organs (32), welches besagten Schwingarm (33) mit besagter Welle mechanisch verbindet, wobei besagtes mechanisches Verbindungsorgan (32) um die erste Achse angeordnet ist;

– Mittel (1001–1002), um eine zweite feste Drehachse (100) des Messwandlers zu erhalten, wobei besagte erste Achse (31) und besagte zweite Drehachse (100) einander im Drehzentrum (0) schneiden und zueinander senkrecht angeordnet sind;

– und einen Bügel (34), der um eine dritte Achse (104) drehbar angeordnet ist, die mit dem Messwandler fest verbunden ist, durch besagten Drehmittelpunkt (0) verläuft und senkrecht zu besagter zweiter Drehachse (100), wobei besagter Bügel fest mit dem zweiten Ende des Schwingarms verbunden ist,

Sonde, bei der die Motorwelle um eine Referenzstellung (320, Figur 5) schwingt, die zweite feste Drehachse (100) des Messwandlers und die dritte Achse (104) des Bügels (34) direkt mit dem piezoelektrischen Element (1) verbunden sind, der Schwingarm (33) an seinem zweiten Ende fest an einem bestimmten Punkt so auf den Bügel (34) montiert ist, dass die Verlängerung der Achse des Schwingarms durch den Drehmittelpunkt (0) verläuft und besagtes mechanisches Verbindungsorgan (32) so angeordnet ist, dass das erste Ende (330), welches im Verhältnis zur ersten Achse (31) verschoben ist, eine kreisförmige Bewegung um besagte erste Achse (31) ausführt und der Schwingarm (33) eine relative Drehung um seine eigene Achse im Verhältnis zu besagtem Verbindungsorgan (32) ausführen kann.

2. Sonde nach Anspruch 1, dadurch gekennzeichnet, dass besagtes mechanisches Verbindungsorgan eine Scheibe (32) enthält, die senkrecht zu besagter Welle (31) angeordnet und fest mit ihr verbunden ist, sowie ein Wälzlager (331–332), in das das erste Ende (330) des Schwingarms (33) eingebaut ist, wobei besagte Scheibe (32) mit einem Magneten (320) ausgestattet ist, der mit festen Magneten (201–202) so zusammenarbeitet, dass ein Rückstellmoment der beweglichen Teile in eine Gleichgewichtsstellung entsteht.

3. Sonde nach Anspruch 1 oder 2, welche ausserdem eine Vorrichtung zur ständigen Positionsbestimmung des Ultraschallstrahls besitzt, dadurch gekennzeichnet, dass besagte Bestimmungsvorrichtung eine Brücke (355–356–350, E) zur Messung der von besagtem Antriebsorgan hervorgebrachten gegenelektromotorischen Kraft besitzt, welches Organ eine von einem Rückstellmoment bestimmte Referenzposition oder -mechanik besitzt und Mittel (39), um das erhaltene Messsignal zu integrieren, wobei besagte Mittel so angeordnet sind (Widerstand 361 – Kondensator 360), dass das Ausgangssignal gleich Null ist, wenn besagtes Antriebsorgan nicht in Bewegung ist.

4. Sonde nach Anspruch 3, gekennzeichnet durch Mittel (Kondensatoren 353 und 354), um die Übermittlung von Strom niederer Frequenz (Generator 35) zur Ansteuerung des Motors an die Sende- (4) und Empfangs- (5) organe des Untersuchungsapparates und den Messwandler (1) zu verhindern und durch Mittel (induktive Widerstände 351 und 352), um die Übermittlung von Hochfrequenz-Ausgangs- und Eingangsstrom an besagte Messbrücke zu verhindern, damit die Sonde durch ein einziges koaxiales Kabel (6) an die äusseren Vorrichtungen angeschlossen werden kann.

5. Sonde nach einem der Ansprüche 3 oder 4, dadurch gekennzeichnet, dass besagte Messbrücke einen Verstärker (364) mit variabler Verstärkung besitzt, welcher von einem synchronen Demodulator (365) gesteuert wird, der einerseits von der Brücke das besagte Ausgangssignal empfängt und andererseits eine Spannung, deren Frequenz hoch genug ist, um nicht den Motor anzutreiben, wobei besagte Spannung an die Brücke angelegt wird.

6. Sonde nach einem der Ansprüche 3 bis 5, mit Mitteln (8–82), um eine Spannung proportional zu besagtem Ausgangssignal zu entnehmen, sie mit einer regulierbaren Gleichspannung zu vergleichen und, bei Übereinstimmung, ein Signal hervorzubringen, welches die Kathodenstrahlröhre des Untersuchungsgerätes zum Leuchten bringt, und Mitteln (83–85–86), um an den Motor eine Spannung proportional zu besagter Gleichspannung anzulegen, welche die Stelle des normalen Motoransteuerstrom einnimmt, um den Motor in einer Winkelstellung anzuhalten, die der von besagtem Signal zur Hervorrufung der Leuchtverstärkung bestimmten entspricht.

7. Sonde nach Anspruch 1, zur Hervorbringung eines Ultraschallstrahles, der in der Untersuchungsebene eine rasche Winkelabtastung ausführt, wobei besagter Strahl an das untersuchte Milieu durch eine Kupplungsflüssigkeit übertragen wird, welche in einer Kammer enthalten ist, durch eine ultraschalldurchlässige Membran (113), die eine Wand dieser Kammer bildet, wobei besagte Kupplungsflüssigkeit so ausgebildet ist, dass die Fortpflanzungsgeschwindigkeit der Ultraschallwellen darin wesentlich verschieden ist von der Fortpflanzungsgeschwindigkeit in Wasser, gekennzeichnet durch eine Hilfskammer, begrenzt von besagter Hauptmembran (112–113) und eine vorformbare Hilfsmembran (114), die Wasser enthält, wobei die beiden Membrane und die Kupplungsflüssigkeit im wesentlichen die gleiche Schallimpedanz haben wie Wasser und mindestens ein Teil (122) der Oberfläche besagter Hauptmembran (112–113) verformbar genug ist, um ein Flüssigkeitsdruckgleichgewicht in den beiden Kammern zu ermöglichen.

8. Sonde nach Anspruch 7, dadurch gekennzeichnet, dass die Hauptmembran einen ersten Teil (113) enthält, der den Boden der Hauptkammer verschliesst und einen zweiten Teil, gebildet durch Abschnitte eines sehr verformbaren Streifens (112), die gegenüber von Fenstern (1110 bis 1113) angeordnet sind, welche in der Seitenwand besagter Hauptkammer angebracht sind.

9. Sonde nach Anspruch 7 oder 8, dadurch gekennzeichnet, dass der Abschnitt (113) der Hauptmembran, welcher den Boden der Hauptkammer verschliesst, so ausgebildet ist, dass er eine Konvexlinse bildet.

10. Sonde nach einem der Ansprüche 7 bis 9, dadurch gekennzeichnet, dass die Seitenwand der Hilfskammer durch einen Stutzen (13) mit Innengewinde an seinem oberen Ende gebildet wird, welcher mit einem Gewindering (12) zusammenarbeitet, welcher die Wasserdruckregulierung in der Hilfskammer ermöglicht.

11. Sonde nach einem der Ansprüche 7 bis 10, gekennzeichnet durch eine geschlossene luftgefüllte Vertiefung (114), die so ausgelegt ist, dass eine Veränderung ihres Volumens je nach der Temperatur eine gegensätzliche Volumenveränderung der Flüssigkeit in der Hauptkammer zur Folge hat.

Fig.1

Fig. 2

Fig. 3

Fig.4

Fig.5

0 045 265

Fig. 6

# Fig.7

Fig. 8

Fig. 9

Fig. 10